(19) Europäisches Patentamt
European Patent Office
Office européen des brevets

(11) Veröffentlichungsnummer: **0 046 542**
**A1**

(12) **EUROPÄISCHE PATENTANMELDUNG**

(21) Anmeldenummer: **81106305.6**

(22) Anmeldetag: **13.08.81**

(51) Int. Cl.³: **C 07 D 307/32**
C 11 B 9/00
//C07D307/60

(30) Priorität: **21.08.80 DE 3031486**

(43) Veröffentlichungstag der Anmeldung:
**03.03.82 Patentblatt 82/9**

(84) Benannte Vertragsstaaten:
**AT BE CH DE FR GB IT LI LU NL SE**

(71) Anmelder: **Henkel Kommanditgesellschaft auf Aktien**
**Postfach 1100 Henkelstrasse 67**
**D-4000 Düsseldorf 1(DE)**

(72) Erfinder: **Upadek, Horst, Dr.**
**Kempenweg 18a**
**D-4006 Erkrath 2(DE)**

(72) Erfinder: **Conrad, Jens, Dr.**
**Dürerweg 15**
**D-4010 Hilden(DE)**

(72) Erfinder: **Bruns, Klaus, Dr.**
**Notburgaweg 6**
**D-4150 Krefeld-Traar(DE)**

(72) Erfinder: **Salz, Rainer, Dr.**
**Itterstrasse 33**
**D-4000 Düsseldorf 13(DE)**

(54) **Neue alpha/beta-substituierte gamma-Butyrolactone, deren Herstellung und Verwendung als Riechstoffe, sowie diese enthaltende Riechstoffkompositionen.**

(57) Beanspruchte sind α/β-substituierte, ungesättigte γ-Butyrolactone der allgemeinen Formel I:

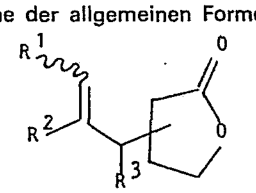

1

$R^1,R^2,R^3$= H oder Alkyl($C_{1-7}$), wobei mindestens einer dieser Reste ein Alkylrest und die Summe der C-Atome in den Resten 1-7 ist, bevorzugt α/β-(2-Decenyl)-γ-butyrolacton.

Zur Herstellung der Verbindungen wird in erster Stufe ein Olefin der Formel II

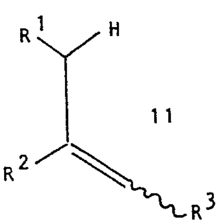

11

($R^1,R^2,R^3$ in vorgenannter Bedeutung) das in Allylposition mindestens ein H-Atom besitzt, mit Maleinsäureanhydrid bei ca. 210-240°C unter Stickstoffatmosphäre im Autoklaven mehrere Stunden erhitzt. Dabei bildet sich in einer En-Reaktion das entsprechende α-(2-Alkenyl)-succinanhydrid. Dieses wird in zweiter Stufe mit Natriumborhydrid zum Gemisch der Strukturisomeren von α/β-substituierten γ-Buturolactonen reduziert (I).

EP 0 046 542 A1

D-4000 Düsseldorf, den 19.8.1980

HENKEL KGaA
ZR-FE/Patente
z-sü

# P a t e n t a n m e l d u n g

D 6156 EP

**"Neue α/β-substituierte γ-Butyrolactone deren Herstellung und Verwendung als Riechstoffe, sowie diese enthaltende Riechstoffkompositionen"**

Es wurde gefunden, daß α/β -substituierte ungesättigte γ-Butyrolactone der allgemeinen Formel

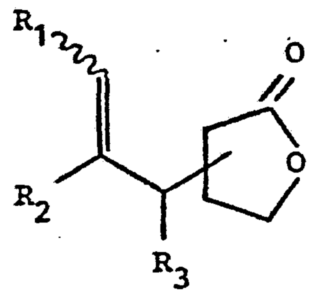

(I) ,

in der $R_1$, $R_2$ und $R_3$ Wasserstoff oder einen Alkylrest mit 1 - 7 C-Atomen darstellen, mit der Maßgabe, daß mindestens einer der Reste $R_1$, $R_2$, $R_3$ ein Alkylrest und die Summe der C-Atome der 3 Reste 1 bis 7 ist, wertvolle neue Riechstoffe darstellen, die insbesondere für neuartige Riechstoffkompositionen geeignet sind.

Die Herstellung der erfindungsgemäßen neuen α/β-substituierten ungesättigten γ-Butyrolactone erfolgte nach an sich bekannten Syntheseverfahren der organischen Chemie in zwei Syntheseschritten. Im ersten Verfahrensschritt wird ein Olefin, das in Allylposition mindestens ein Wasserstoffatom besitzt, mit Maleinsäureanhydrid bei einer Temperatur von circa 210 - 240°C unter Stickstoffatmosphäre mehrere Stunden im Autoklaven erhitzt. Dabei bildet sich in einer En-Reaktion das entsprechende α-(2-Alkenyl)-succinanhydrid. Die Reaktion verläuft dabei nach folgendem Schema, wobei $R_1$, $R_2$ und $R_3$ die vorgenannte Bedeutung besitzen:

...

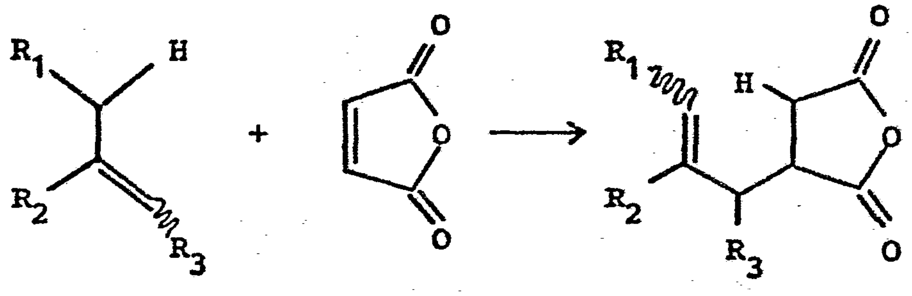

(II)

Anschließend wird dann in zweiter Stufe das α-(2-Alkenyl)-succinanhydrid (II) mit Natriumborhydrid zum Gemisch der strukturisomeren α/β-substituierten γ-Buturolactone (III-VI) nach folgendem Schema reduziert:

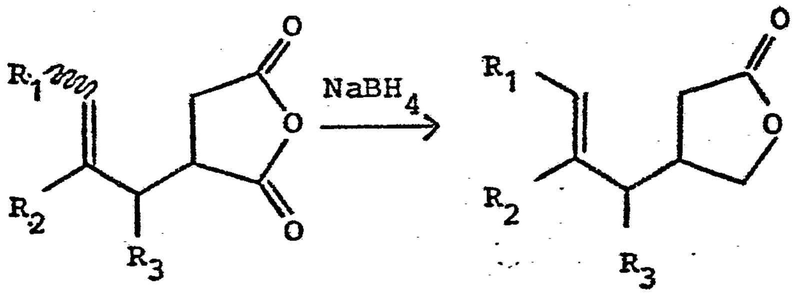


(III)

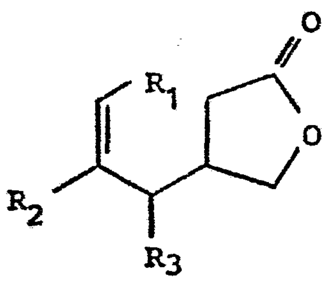

(IV) +

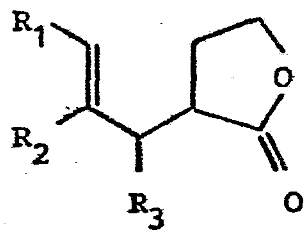

(V)

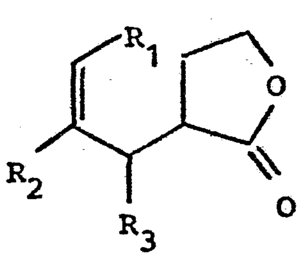

(VI)

...

Beim Einsatz von unsymmetrisch substituierten Olefinen, die zum Beispiel in zwei verschiedenen Allylpositionen zur En-Reaktion befähigte Wasserstoffatome tragen, resultieren gemäß dem Herstellungsschema insgesamt zwei mal vier Strukturisomere.

Das erhaltene Lactongemisch entspricht den unter die Formel (I) fallenden Verbindungen und kann ohne weitere Auftrennung als Riechstoff verwendet werden.

Zur vollständigen Reduktion des α-(2-Alkenyl)-succinanhydrids ist ein Überschuß an Natriumborhydrid erforderlich. Als inertes Lösungsmittel für das Reaktionsgut sind zum Beispiel niedere Alkohole wie Ethanol oder Isopropanol geeignet. Um eine Weiterreduktion über die Lactonstufe hinaus zu den entsprechenden 1,4-Diolen zu verhindern, wird die Reduktion bei Zimmertemperatur oder bei schwach erhöhter Temperatur bis maximal circa 60°C durchgeführt.

Es ist bereits von anderen Reduktionen von Anhydriden zu Lactonen bekannt, daß diese Reaktion auch durch katalytische Hydrierung möglich ist. Bei der katalytischen Hydrierung werden jedoch in der Regel olefinische Doppelbindungen mithydriert. Ein Vergleich der durch Hydrierung mit Natriumborhydrid hergestellten ungesättigten Lactone der Formel (I) mit den durch katalytische Hydrierung über Raney-Nickel hergestellten, entsprechenden gesättigten Lactone ergab, daß die Hydrierung der Doppelbindung einen ungünstigen Effekt auf die Geruchsqualität der Produkte hat. Der für die Erzielung einer guten Riechstoffqualität erforderliche vollständige Erhalt der Doppelbindung ist bei Einsatz von Natriumborhydrid als Reduktionsmittel gewährleistet.

...

Die neuen Riechstoffe, die ein Gemisch der strukturisomeren Verbindungen (III bis VI) darstellen und der allgemeinen Strukturformel (I) entsprechen, zeichnen sich durch kräftige und anhaftende Geruchsnoten aus und sind mit großem Vorteil zu neuartigen Kompositionen kombinierbar, denen sie gleichfalls eine gute Haftfestigkeit und interessante Geruchsnuancen verleihen.

Als erfindungsgemäße neue Riechstoffe sind zum Beispiel α/β-(2-Hexenyl)-, α/β-(2-Octenyl)-, α/β-Isooctenyl-, α/β-(2-Decenyl)-, α/β-(1,2-Dimethyl-2-propenyl)-, α/β-(2-Methyl-2-butenyl)-, α/β-(2-Ethyl-2-propenyl)-γ-butyrolacton zu nennen. Unter diesen kommt wiederum dem α/β-(2-Decenyl)-γ-buturolacton in parfümistischer Hinsicht die größte Bedeutung zu, da es sich durch eine frische,an Mandarinenschalen und nasses Hundefell erinnernde Geruchsnote sowie durch eine besonders große Haftfestigkeit auszeichnet, die es in damit hergestellten Kompositionen auch auf diese überträgt.

Die erfindungsgemäßen α/β-substituierten ungesättigten γ-Butyrolactone können mit anderen Riechstoffen in den verschiedensten Mengenverhältnissen zu neuen Riechstoffkompositionen gemischt werden. Im allgemeinen wird sich ihr Anteil in den Riechstoffkompositionen in den Mengen von 1 bis 50 Gewichtsprozent, bezogen auf die gesamte Komposition bewegen. Derartige Kompositionen können zur Parfümierung von kosmetischen Präparaten wie Cremes, Lotionen, Duftwässern, Aerosolen, Toiletteseifen als auch in der Extrait-Parfümerie verwendet werden. Sie können aber auch zur Geruchsverbesserung technischer Produkte wie Wasch- und Reinigungsmittel, Weichspüler, Textilbehandlungsmittel eingesetzt werden. Zur Parfümierung der verschiedenen Produkte werden diesen die Kompositionen im allgemeinen in Konzentrationen von 0,05 bis 2 Gewichtsprozent, bezogen auf das gesamte Produkt, zugesetzt. ...

Die nachfolgenden Beispiele sollen den Gegenstand der Erfindung näher erläutern, ohne ihn jedoch hierauf zu beschränken.

...

## B e i s p i e l e

1. α/β-(2-Decenyl)-γ-butyrolacton

1. Stufe:

421 g (3 Mol) 1-Decen, 196 g (2 Mol) Maleinsäureanhydrid und 3 g Hydrochinon wurden unter Stickstoffatmosphäre 4 Stunden lang bei 230 °C im Autoklaven erhitzt. Durch Vakuumdestillation wurden aus dem Reaktionsprodukt bei 136 - 146 °C unter einem Druck von 0,01 mbar 315 g, das sind 66 % der theoretischen Ausbeute an α-(2-Decenyl)-succinanhydrid erhalten.

2. Stufe:

Zu 64,2 g (1,7 Mol) Natriumborhydrid in 950 ml Isopropanol wurden unter Rühren 310 g (1,3 Mol) α-(2-Decenyl)-succinanhydrid zugetropft. Es wurde circa 20 Stunden lang bei Raumtemperatur nachgerührt, das Isopropanol im Vakuum abdestilliert und der weiße Rückstand mit einer Lösung von 580 ml konzentrierter Salzsäure in 1550 ml Wasser versetzt. Hernach wurde noch circa 2 Stunden bei Raumtemperatur gerührt, mit Ether extrahiert, die vereinigten organischen Phasen wurden neutral gewaschen und nach Abdestillieren des Ethers wurde das erhaltene Rohprodukt im Vakuum destilliert. Es wurden 238 g α/β-(2-Decenyl)-γ-butyrolacton, das sind 88 % der theoretischen Ausbeute, erhalten. Die neue Verbindung besaß folgende Kenndaten:

Kp 118-122 °C / 0,04 mbar $n_D^{20}$ = 1,4661
IR (Film): 1780 $cm^{-1}$ (5-Ring-Lactonbande)
$^1$H-NMR ($CDCl_3$): u. a. δ = 5,2 - 5,8 (m, 2H, olefinische H)
3,8 - 4,6 (m, 2H, $-CH_2O-$)

...

Das Produkt besitzt einen intensiven und festhaftenden Geruch nach Mandarinenschalen und nassem Hundefell.

In analoger Weise wurden die nachfolgend aufgeführten Lactone hergestellt:

2. <u>α/β-(2-Hexenyl)-γ-butyrolacton</u>

Kp. 94°C/0,07 mbar $n_D^{20}$ = 1,4642
Geruch: Kokos-, Cord-Note

3. <u>α/β-(2-Octenyl)-γ-butyrolacton</u>

Kp. 101°C / 0,02 mbar $n_D^{20}$ = 1,4652
Geruch: Brackwasser-,Madarinen-Note

4. <u>α/β-Isooctenyl-γ-butyrclacton</u>
Die Herstellung erfolgte gemäß Stufe 2 aus dem im Handel erhältlichen Isooctenylsuccinanhydrid.
Kp. 125°C/4 mbar $n_D^{20}$ = 1,4666
Geruch: ledrig, Castoreum-Note

5. <u>α/β-(1,2-Dimethyl-2-propenyl)-γ-butyrolacton</u>

Kp. 62°C/0,01 mbar $n_D^{20}$ = 1,4723
Geruch: Leder-, Castoreum-, Birkenteer-Note

6. Strukturisomerengemisch aus
α/β-(2-Methyl-2-butenyl)- und
α/β-(2-Ethyl-2-propenyl)-γ-butyrolacton.

Kp. 111°C/ 6 mbar $n_D^{20}$ = 1,4715
Geruch: Irotyl-, Lumberjack-Note

...

7. Phantasie-Eau de Cologne

| | | |
|---|---|---|
| α/β-(2-Decenyl)-γ-butyrolacton | 100 | Gewichtsteile |
| Bergamottöl | 355 | " |
| Citronenöl | 215 | " |
| Orangenöl süß | 145 | " |
| Petitgrainöl | 145 | " |
| Neroliöl | 40 | " |

8. Ambron-Komplex

| | | |
|---|---|---|
| α/β-(2-Decenyl)-γ-butyrolacton | 120 | Gewichtsteile |
| α/β-Isooctenyl-γ-butyrolacton | 30 | " |
| Bergamottöl | 110 | " |
| Res. Labdanum | 100 | " |
| Res. Benzoe | 100 | " |
| Heliotropin | 90 | " |
| Isobutylcinnamat | 80 | " |
| Jasmin künstlich | 70 | " |
| Ketonmoschus | 65 | " |
| Patchouliöl | 40 | " |
| Cypressenöl | 30 | " |
| Vetiveröl | 10 | " |
| Diethylphthalat | 130 | " |

...

Patentansprüche:

1. α/β-substituierte ungesättigte γ-Butyrolactone der allgemeinen Formel

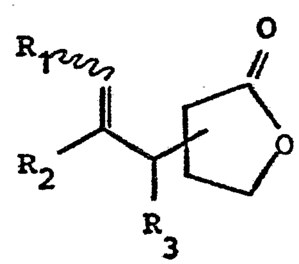

in der $R_1$, $R_2$, und $R_3$ Wasserstoff oder einen Alkylrest mit 1 - 7 C-Atomen darstellen, mit der Maßgabe, daß mindestens einer der Reste $R_1$, $R_2$, $R_3$ ein Alkylrest und die Summe der C-Atome der drei Reste 1 - 7 ist.

2. α/β-(2-Decenyl)-γ-butyrolacton.

3. Verfahren zur Herstellung der α/β-substituierten ungesättigten γ-Butyrolactone, bei dem man in erster Stufe ein Olefin der allgemeinen Formel

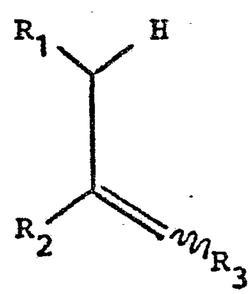

in der $R_1$, $R_2$, $R_3$ die vorgenannte Bedeutung besitzen, mit Maleinsäureanhydrid bei einer Temperatur von circa 210 - 240°C unter Stickstoffatmosphäre im Autoklaven umsetzt und das erhaltene α-(2-Alkenyl)-

...

succin-anhydrid in zweiter Stufe mit einem Überschuß an Natriumborhydrid in einem inerten Lösungsmittel bei Zimmertemperatur oder schwach erhöhter Temperatur bis maximal circa 60 °C zum α/β-substituierten ungesättigten γ-Butyrolacton reduziert.

4. Verwendung der α/β-substituierten ungesättigten γ-Butyrolactone nach Anspruch 1 und 2 als Riechstoffe.

5. Riechstoffkompositionen, dadurch gekennzeichnet, daß sie die α/β-substituierten ungesättigten γ-Butyrolactone in einer Menge von 1 - 50 Gewichtsprozent bezogen auf die gesamte Komposition, enthalten.

0046542

Europäisches Patentamt

**EUROPÄISCHER RECHERCHENBERICHT**

Nummer der Anmeldung

EP 81 10 6305

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| X | JOURNAL OF ORGANIC CHEMISTRY, Band 44, Nr. 13, veröffentlicht in 1979 EASTON OHIO (USA) W.H. PIRKLE et al. "Broad-spectrum synthesis of enantiomerically pure lactones", Seiten 2169-2175<br>* Formel 3,4; Seite 2171, linke Spalte *<br>-- | 1-2,4, 5 | C 07 D 307/32<br>C 11 B 9/00//<br>C 07 D 307/60 |
| X | CHEMICAL ABSTRACTS, Band 87, Nr. 21, veröffentlicht am 21. November 1977, Seite 502-3, Zusammenfassung 167125c COLUMBUS OHIO (US) & Neftekhimiya 1977, 17(4) 573-5 E.I. RUBINSHTEIN et al "Condensation of maleic anhydride with olefins"<br>* Zusammenfassung *<br>-- | 3 | RECHERCHIERTE SACHGEBIETE (Int. Cl.³)<br>C 07 D 315/00<br>307/32<br>C 11 B 9/00 |
| X | S. PATAI "The chemistry of acid derivatives", Supplement B Teil 2 J. WILEY & SONS NEW YORK (US) J.F. WOLFE et al "By reduction of anhydrides, esters and acids" Seite 1138<br>* Seite 1138, letzter Absatz *<br>-- | 3 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| | US - A - 4 011 177 (H. RAHMAN ANSARI)<br>* das ganze Dokument *<br>-- ./. | 1,4,5 | |

X Der vorliegende Recherchenbericht wurde für alle Patentansprüche erstellt.

| Recherchenort | Abschlußdatum der Recherche | Prüfer |
|---|---|---|
| Den Haag | 24-11-1981 | NUYTS |

Europäisches Patentamt

# EUROPÄISCHER RECHERCHENBERICHT

Nummer der Anmeldung
EP 81 10 6305


| EINSCHLÄGIGE DOKUMENTE | | | KLASSIFIKATION DER ANMELDUNG (Int Cl 3) |
|---|---|---|---|
| Kategorie | Kennzeichnung des Dokuments mit Angabe, soweit erforderlich, der Maßgeblichen Teile | betrifft Anspruch | |
| | CH - A - 586 179 (GIVAUDAN)<br>* Spalte 3, Zeile 64 - Spalte 4, Zeile 23 *<br>----------- | 1,4,5 | |
| | | | RECHERCHIERTE SACHGEBIETE (Int. Cl.3) |